(19) Europäisches Patentamt / European Patent Office / Office européen des brevets

(11) EP 3 795 151 A1

(12) EUROPEAN PATENT APPLICATION
published in accordance with Art. 153(4) EPC

(43) Date of publication:
**24.03.2021 Bulletin 2021/12**

(21) Application number: **19791524.2**

(22) Date of filing: **19.04.2019**

(51) Int Cl.:
***A61K 31/385** (2006.01)*
***A61K 31/795** (2006.01)*
***A61K 47/34** (2017.01)*
***A61K 9/127** (2006.01)*
***A61P 35/00** (2006.01)*

(86) International application number:
**PCT/CN2019/083390**

(87) International publication number:
**WO 2019/206030 (31.10.2019 Gazette 2019/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.04.2018 CN 201810380857**

(71) Applicant: **Sichuan Yuanning Biological Technology Co., Ltd.**
**Industrial Development Zone**
**Pengzhou, Sichuan 611900 (CN)**

(72) Inventors:
- **ZHANG, Shiyong**
  **Chengdu, Sichuan 610041 (CN)**
- **LIAO, Chunyan**
  **Chengdu, Sichuan 610041 (CN)**
- **CHEN, Ying**
  **Chengdu, Sichuan 610041 (CN)**
- **DAI, Xin**
  **Chengdu, Sichuan 610041 (CN)**

(74) Representative: **Crease, Devanand John et al**
**Keltie LLP**
**No.1 London Bridge**
**London SE1 9BA (GB)**

# (54) ANTITUMOR NANO MEDICAMENT

(57) An antitumor nano-drug, a preparation method and use thereof, wherein the nano-drug mainly takes lipoic acid polymer as an active component. The nano-drug can reduce toxic and side effects, and can be used in combination with other anti-tumor active substances.

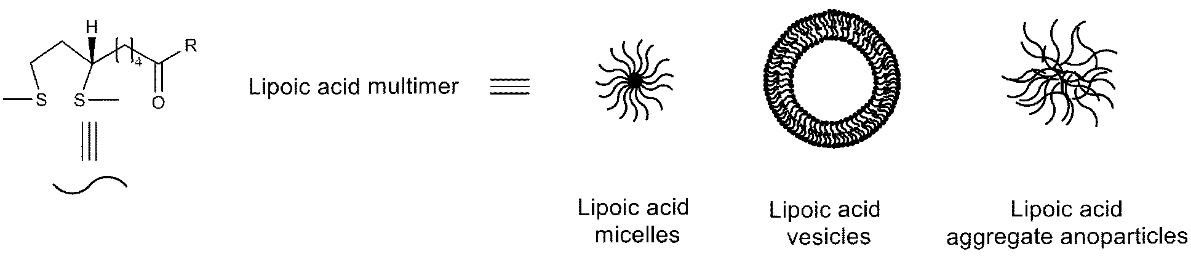


FIG. 1

EP 3 795 151 A1

## Description

### CROSS REFERENCE TO RELATED APPLICATION

This application claims the priority of Chinese Patent Application CN201810380857.9, entitled "antitumor nano-drug" filed with the Chinese Patent Office on April 25, 2018, which is incorporated herein by reference in its entirety.

### TECHNICAL FIELD

The disclosure belongs to the field of biomaterials, particularly relates to an antitumor nano-drug.

### BACKGROUND ART

Cancer is a kind of common and multiple major diseases that seriously endanger human health, and its mortality rate accounts for the second place among all human diseases. Chemotherapy is an important means for cancer treatment, but traditional chemotherapeutic drugs can easily cause toxic and side effects such as impaired liver and kidney function, myelosuppression and decreased human immunity. Therefore, developing new chemotherapeutic drugs with antitumor activity but with no or low toxicity to normal tissues can well solve the problems of traditional chemotherapeutic drugs.

(R)-(+)-lipoic acid (LA) is a kind of B vitamins synthesized by lipoic acid synthase in mitochondria, which has the functions of stabilizing blood sugar, strengthening liver function, relieving fatigue, beautifying skin and anti-aging. In addition, it has been found that a large dose of LA has a certain antitumor effect but has non-toxic to normal cells at the same dose. So, LA has an excellent application prospect as a natural antitumor active substance. However, due to the hydrophilicity and lipophilicity of the small molecule LA, it can reach any tissues after entering the body and is easy to be quickly removed, resulting in a large dosage and poor efficacy. The drug combination can improve the therapeutic effect of LA, but when combined with other small molecule drugs, they can not enter the cell in a predetermined ratio, making it difficult to achieve the effect of " 1+1 > 2".

### SUMMARY OF THE INVENTION

To solve the above problems, a novel antitumor nano-drug is developed in the disclosure. (*R*)-(+)-lipoic acid is prepared into lipoic acid multimer that is loaded on a nano-drug carrier or directly prepared into nanoparticles, which significantly improves the antitumor effect of LA monomer and avoids the toxic and side effects of traditional chemotherapy drugs. Moreover, the antitumor active substance of lipoic acid multimer can be used in combination with other antitumor active substances, which can enter cells in a predetermined ratio, so that a synergistic antitumor effect of " 1+1 > 2" can be achieved and the curative effect of nano-drug will be further improved.

The disclosure is realized through the following technical schemes:

An antitumor nano-drug, wherein the antitumor active ingredient mainly exists in the form of lipoic acid multimer.

Alternatively, the nano-drug can exist in the form of liposomes, dendrimers, polymer micelles, vesicles, aggregates and the like.

Alternatively, as in the above antitumor nano-drug, the lipoic acid multimer contains an R chiral structure.

Alternatively, as in the above antitumor nano-drug, the lipoic acid multimer is mainly constructed from (*R*)-(+)-lipoic acid or pharmaceutically acceptable salt thereof.

Alternatively, as in the above antitumor nano-drug, the structural formula of lipoic acid multimer is as follows:

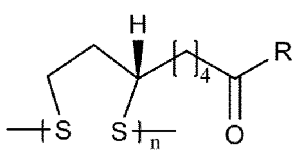

Wherein, n ≥ 2, R is hydroxyl group or functional group connected by ester bond/amide bond or $O^-M^+$ structure ($M^+$ is metal ion).

Alternatively, as in the above antitumor nano-drug, the lipoic acid multimer exists in at least one of chain polymers, micelles, vesicles and aggregates.

Alternatively, as in the above antitumor nano-drug, the lipoic acid multimer is prepared by lipoic acid itself or with the aid of template molecules.

Alternatively, as in the above antitumor nano-drug, the lipoic acid multimer can be used in combination with other antitumor active substances.

Alternatively, as in the above antitumor nano-drug, the lipoic acid multimer can cooperate with other antitumor active substances to suppress tumor growth, which has the synergistic effect of" 1+1 > 2".

Alternatively, as in the above antitumor nano-drug, the other antitumor active substances are any substances that can produce synergistic antitumor effect with the lipoic acid multimer. Specifically including: traditional chemotherapy small molecule drugs, such as camptothecin, hydroxyurea, pixantrone, doxorubicin hydrochloride, gemcitabine hydrochloride, cytarabine, etc; natural antitumor active substances, such as anthocyanin, resveratrol, curcumin, tomatin, tea polyphenol, astaxanthin and the like.

Alternatively, as in the above antitumor nano-drug, the lipoic acid multimer can be physically loaded on or connected by chemical bond to the nano-carrier. The nano-carriers can be liposomes, dendrimers, polymer micelles, vesicles, aggregates, lipoic acid polymers, etc.

Alternatively, as in the above antitumor nano-drug, the lipoic acid multimer also act as a nano-carrier. The lipoic acid chain polymers, micelles, vesicles or aggregates can be directly used for antitumor.

Alternatively, as in the above antitumor nano-drug, the lipoic acid multimer can be physically loaded on or connected by chemical bond to the nano-carrier, while the nano-carrier can also load other antitumor active substances, both of which cooperate with each other in antitumor; or the lipoic acid multimer directly load the other antitumor active substances, both of which cooperate with each other in antitumor.

Alternatively, as in the above antitumor nano-drug, the lipoic acid multimer can be physically loaded on dendrimer for antitumor.

Alternatively, as in the above antitumor nano-drug, the lipoic acid multimer can be physically loaded on liposome for antitumor.

Alternatively, as in the above antitumor nano-drug, the lipoic acid multimer can be physically loaded on liposome. Meanwhile, the liposome loads a hydrophobic natural antitumor active substance- curcumin.

Alternatively, as in the above antitumor nano-drug, the lipoic acid multimer can be physically loaded on liposome. Meanwhile, the liposome loads a hydrophilic chemotherapeutic drug- gemcitabine hydrochloride.

Alternatively, as in the above antitumor nano-drug, the lipoic acid multimer can be physically loaded on liposome. Meanwhile, the liposome loads a hydrophilic natural antitumor active substance- resveratrol.

Alternatively, as in the above antitumor nano-drug, the lipoic acid multimer in the form of chain polymers, micelles, vesicles or aggregates can physically load or covalently connect the other antitumor active substances.

Alternatively, as in the above antitumor nano-drug, the lipoic acid micelle can covalently connect the hydrophilic chemotherapeutic drug-cytarabine, while physically loads the hydrophobic natural antitumor active substance-anthocyanin.

Alternatively, as in the above antitumor nano-drug, the lipoic acid vesicle can physically load hydrophilic chemotherapeutic drug-hydroxyurea.

Alternatively, as in the above antitumor nano-drug, the lipoic acid vesicle can covalently connect the hydrophilic chemotherapeutic drug-cytarabine, while physically loads the hydrophilic chemotherapeutic drug-hydroxyurea.

Alternatively, as in the above antitumor nano-drug, LA can covalently connect with other antitumor active substances, then the obtained compound is constructed into chain polymers, micelles, vesicles, aggregates and the like.

Alternatively, as in the above antitumor nano-drug, LA is directly covalently connect with the hydrophilic drug-hydroxyurea,, which is used to directly form micelles, and further cross-linked by lipoic acid to form stable nano-particles.

Alternatively, as in the above antitumor nano-drug, LA can be physically mixed with other antitumor active substances to form chain polymers, micelles, vesicles, aggregates and the like.

Alternatively, as in the above antitumor nano-drug, LA can directly form aggregates with the chemotherapeutics-camptothecin and natural antitumor active substance-anthocyanins, and further forms stable nanoparticles through lipoic acid cross-linking.

The disclosure also provides a application of the lipoic acid multimer, wherein the lipoic acid multimer can be used for preparing the antitumor nano-drug.

All features disclosed in this description, or all disclosed methods or steps in the process, except for mutually exclusive features and/or steps, can be combined in any manner.

Benificial effect of the disclosure:

The nano-drug in the disclosure selects non-toxic lipoic acid multimer as an antitumor active ingredient, which can avoid the toxic and side effects caused by traditional chemotherapy drugs, overcome the inherent defects of small molecular drugs, are difficult to be removed after entering the blood circulation, and have good targeting property. Therefore, nano-drug hold a higher antitumor activity than lipoic acid monomers. When lipoic acid multimer is used in combination with other antitumor active substances, it can also enter the cell at a predetermined ratio. Compared with the simple mixture of several drugs, nano-drug can achieve a synergistic antitumor effect of " 1+1 > 2" due to the controllable ratio of combined drugs, thus further improving the efficacy of nano-drug.

## BRIEF DESCRIPTION OF THE DRAWINGS

FIG. 1 is the schematic diagram of lipoic acid multimer in Example 1;

FIG. 2 is the characterization result diagram of micelles, vesicles, and nanoparticles of lipoic acid in Example 1, wherein a) is a DLS dimensional drawing, b) and c) show the fluorescence intensity changes of Phloxine B before and after Triton X-100 was added after lipoic acid vesicles and lipoic acid aggregates coated with Phloxine B passed through gel column;

FIG. 3 is a graph showing the antitumor mechanism of lipoic acid micelles in Example 2, wherein a) is the toxicity test results of lipoic acid micelles on human colon cancer cells (SW480), b) is the relative $O_2^{-\cdot}$ level in mitochondria after treated with different materials, c) is the percentage of mitochondrial membrane potential loss after treated with different materials, and d) is the relative Caspase-3 activation amount after treated with different materials;

FIG. 4 shows the cytotoxicity test result of lipoic acid micelles and lipoic acid monomers in Example 3;

FIG. 5 shows the cytotoxicity test results of nano-drug I and nano-drug II in Example 4.

FIG. 6 shows the cytotoxicity test results of lipoic acid micelles on normal cells in Example 5, wherein a) is the test result on human kidney epithelial cells (293T), and b) is the test results on mouse fibroblasts (3T3);

FIG. 7 shows the evaluation of the hemolytic and coagulant properties of lipoic acid micelles in Example 5, wherein a) is the hemolytic rate, and b) is the coagulant property;

FIG. 8 shows the weight change of mouse in Example 5;

FIG. 9 shows the toxicity assessment of lipoic acid micelles and traditional chemotherapeutic drug molecules on human gingival fibroblasts (HGF) in Example 6, wherein a) is lipoic acid micelles, and b) is gemcitabine hydrochloride and doxorubicin hydrochloride;

FIG. 10 shows the toxicity assessment of three nano-drugs on human gingival fibroblasts (HGF) in Example 6, wherein a) is nano-drug **III,** and b) is nano-drug **IV** and nano-drug **V;**

FIG. 11 shows the toxicity assessment of nano-drug prepared by respectively loading lipoic acid micelle and lipoic acid monomer with curcumin in liposome in Example 7, wherein a) is cytotoxicity and b) is synergism index;

FIG. 12 shows the combined anti-tumor research result of lipoic acid micelles covalently linking with cytarabine in Example 8, wherein a) is cytotoxicity test results and b) is synergism index;

FIG. 13 shows the combined anti-tumor research result of nano-drug prepared by covalently linking lipoic acid with hydroxyurea in Example 9 , wherein a) is cytotoxicity and b) is synergism index;

FIG. 14 shows the combined anti-tumor research result of nanoparticles that directly prepared from lipoic acid, anthocyanin and lycopene in Example 10, where a) is cytotoxicity, and b) is synergism index;

FIG. 15 shows the combined anti-tumor research results of lipoic acid vesicles physically loaded with hydroxyurea in Example 11, wherein a) is cytotoxicity, and b) is synergism index;

FIG. 16 shows the synergistic anti-tumor results of hydroxyurea and lipoic acid vesicles in Example 12, wherein a) is the relative $O_2^{-\cdot}$ level in mitochondria after treated with different materials, b) is the percentage of mitochondrial membrane potential loss, c) is the relative Caspase-3 activation percentage;

FIG. 17 shows the *in vivo* antitumor assessment of nano-drug in Example 13, wherein a) is the tumor volume change of mice, b) is the weight change of mice, c) is the survival rate of mice, and d) is the combination index (Q) of nano-drug *in vivo* synergistic antitumor effect;

FIG. 18 is the schematic structural diagram of the antitumor nano-drug according to the disclosure.

## DETAILED DESCRIPTION OF THE EMBODIMENTS

The above contents of the disclosure are further explained in detail by the specific embodiments of the examples below. However, it should not be understood as limiting the scope of the disclosure to the following examples. Any modifications made without departing from the spirit and principles of the disclosure, and any equivalent substitutions or improvements made according to the general technical knowledge and conventional means in the field, shall be included in the scope of the disclosure. The raw materials used in the following specific examples are available from the market, and the lipoic acid monomers involved in the embodiments are (*R*)-(+)-lipoic acid unless otherwise specified. Percentages mentioned in the examples are percentages by weight unless otherwise specified.

EXAMPLE 1

**Preparation of lipoic acid multimer**

lipoic acid multimer can exist in the form of lipoic acid micelles, lipoic acid vesicles, and lipoic acid nanoparticles, as shown in FIG. 1.

1. Preparation of cross-linked lipoic acid micelles:

(*R*)-(+)-lipoic acid (LA, 100 mg) was added into 50 mL of deionized water, and 1 M NaOH aqueous solution was added dropwise with stirring constantly until the lipoic acid was completely dissolved. Then 1 M HCl solution was added to neutralize the solution. Finally, the solution was freeze-dried to obtain lipoic acid sodium powder which was light yellow. 41.2 mg of lipoic acid sodium (0.2 mmol) was weighted and dissolved in 1 mL of deionized water, and nanoparticles with a size of about 15 nm was obtained after ultrasound. The above obtained nanoparticles were irradiated by the 365 nm ultraviolet light to induce lipoic acid disulfide self-crosslinking. After 2.5 h of reaction and 48 h of dialysis, cross-linked lipoic acid micelles (cLAMs) with a size of about 15 nm were obtained, as shown in FIG. 2a.

2. Preparation of cross-linked lipoic acid vesicles (cLAVs):

122.6 mg of LA (0.6 mmol) and 25.8 mg of template molecule 1,4,7-triazanonane (0.2 mmol) were dissolved in 1 mL of N, N-dimethylformamide (DMF) to obtain a precursor solution with a concentration of 0.2 M, which was shaked for 2 h to form superamphiphilic (SA) molecule mother solution. 50 μL mother solution was added into 5 mL deionized water under ultrasonic condition to prepare vesicle nanoparticles composed of lipoic acid and 1,4,7- triazanonane. Its size detected by DLS, which is about 220 nm.

The above vesicle structure was determined by Phloxine B (PhB) leakage assay. Details were as follows: 50 μL of the SA mother solution was added to 5 mL of a Phloxine B aqueous solution ([PhB] = 0.5 mg/mL) under ultrasonic condition to obtain a nanoparticle solution containing Phloxine B. The obtained solution was separated by a gel column to collect the solution which has Tyndall phenomenon. 40 μL of 10% Triton X-100 solution was added into 2 mL of the obtained collection solution, and the fluorescence intensity change of Phloxine B was monitored before or after adding the Triton X-100. As shown in FIG. 2c, it was found that the fluorescence intensity of Phloxine B was low before the addition of Triton X-100, and the fluorescence intensity was high after adding Triton X-100. This is mainly because the nanoparticles containing a large amount of hydrophilic Phloxine B, which was quenched by fluorescence aggregation at a high concentration, so the fluorescence intensity was very low. However, the structure of nanoparticles is destroyed due to the addition of Triton X-100, which released the contained Phloxine B, thus releasing fluorescnce. Therefore, it was proved to be the vesicles.

The above prepared nanoparticles were irradiated by the 365 nm ultraviolet light to induce lipoic acid disulfide self-crosslinking. After reaction for 2.5 h, the solution was adjusted to alkalinity, and extracted with dichloromethane ($CH_2Cl_2$) to remove the 1,4,7-triazanonane in the solution. After that, the solution was adjusted back to neutral again. After 48 h of dialysis, cross-linked lipoic acid vesicles (cLAVs) with a size of about 220 nm were finally obtained, which was shown in FIG. 2b.

3. Preparation of cross-linked lipoic acid nanoparticles (cLANPs):

41.2 mg of LA was dissolved in 1 mL N,N-dimethylformamide, then the solution was shaked for 2 h to obtain a LA mother solution with a concentration of 0.2 mmol. 50 μL of obtained mother solution was added into 5.0 mL deionized water to obtain lipoic acid nanoparticles with a size of about 80 nm. The obtained nanoparticles were irradiated by the

365 nm ultraviolet light to induce lipoic acid disulfide self-crosslinking. After 2.5 h of reaction and 48 h of dialysis, cross-linked lipoic acid nanoparticles with a size of about 75 nm were obtained, as shown in FIG. 2a.

The structure of the obtained nanoparticles was determined by Phloxine B leakage experiment. Details were as follows: 50 μL of SA mother solution was added to 5 mL of Phloxine B (PhB) aqueous solution ([PhB] = 0.5 mg/mL) under ultrasonic condition to obtain a nanoparticle solution containing Phloxine B. The obtained solution was separated by gel column to collect the solution which has Tyndall phenomenon. 40 μL of 10% Triton X-100 solution was added into 2 mL of obtained collection solution, and the fluorescence intensity change of Phloxine B was monitored before or after the Triton X-100 was added. As shown in FIG. 2d, it was found that the fluorescence intensity of Phloxine B did not change significantly before or after the structure of the nanoparticle was destroyed. Considering its large size, it was judged that the nanoparticles exist in the form of aggregates.

## EXAMPLE 2

### The antitumor mechanism study of the lipoic acid multimer

Choosing cross-lipoic acid micelles (cLAMs) as an example, the antitumor mechanism of lipoic acid multimer was studied.

1. Cytotoxicity assessment:

Human colon cancer cells (SW480) in logarithmic growth active phase were selected and inoculated in 96-well plate. After incubation for 24 h, different concentrations of LA and cLAMs were added into the plate respectively, 5 parallel samples were set for each concentration, and untreated cells were set as blank control group. After incubation for 48 h, the culture medium was removed, and 100 μL fresh medium containing 10% (v/v) MTT was added to each well, and the plates were incubated for another 2 h. Then the culture medium was removed and 150 μL DMSO was added into each well, and the plates were shaked on the oscillator for 2 min. Finally, the absorbance of the solution was measured at 490 nm by using microplate reader. Cell viability was calculated according to the following formula: cell viability (%) = $A_{490}$ (sample)/$A_{490}$ (control) × 100%. As shown in FIG. 3a, it was found that cLAMs have certain cytotoxicity to tumor cell SW480 and show better antitumor activity than LA.

2. Research of antitumor mechanism:

Studies have shown that LA can be partially reduced to dihydrolipoic acid (DHLA) by reduced glutathione (GSH) and thioredoxin reductase (TrxR) after entering into tumor cells, wherein LA can directly oxidize the sulfhydryl group of the protein on the permeability transition pore (mPTP) of the mitochondrial membrane, and DHLA can also indirectly oxidize the sulfhydryl group of the protein on mPTP through the superoxide anion radical ($O_2^{-\cdot}$), both of which can work together to change the permeability of the mitochondrial membrane, resulting in the release of Apoptosis Inducing Factor (AIF) from the mitochondria. The released AIF enters the nucleus and causes DNA agglutination to induce cell apoptosis. At the same time, cytochrome C released from mitochondria can stimulate the expression of pro-apoptotic protein (Caspase-9, Caspase-3) and induce cell apoptosis. In addition, $O_2^{-\cdot}$ produced by DHLA can also reduce the expression of anti-apoptotic proteins Bcl-2 and Bcl-XL, and further increase the expression of pro-apoptotic protein (Caspase-9, Caspase-3), and induce cell apoptosis.

Because cLAMs is constructed by LA, on the one hand, they have the same disulfide bond structure; on the other hand, under the action of intracellular glutathione (GSH) and thioredoxin reductase (TrxR), the reduction product of cLAMs and LA are both DHLA. Based on the same intracellular transformation mode, a series of experiments are designed to verify the anti-tumor mechanism of cLAMs according to the inspection node of the anti-tumor mechanism of LA. The results show that cLAMs can produce $O_2^{-\cdot}$ in the mitochondria, stimulate the opening of the permeability transition pore of the mitochondrial membrane, and cause the decrease of the membrane potential, and further release the cytochrome C from the mitochondria into the cytoplasm, thus regulating the Caspase-3-dependent apoptosis pathway and inducing cell apoptosis, and its antitumor activity is enhanced to some extent compared with LA. The specific experiments are as follows:

Determination of superoxide anion ($O_2^{-\cdot}$) in mitochondria: SW480 cells in logarithmic growth active phase were prepared into cell suspension and added into 6-well plate ($5\times10^5$ cells/well). After incubation for 24 h, cells were treated with 1 mM cLAMs and 1 mM LA, and untreated cells were set as blank control group. After incubation for 4 h, the old culture medium was removed and 1 mL of fluorescent amine dye (50 μM) was added. After incubation for 2 h, cysteine was added to make the final concentration to be 200 μM. After incubation for 0.5 h, culture medium was removed and cells were washed with Krebs buffer for 3 times, then mitochondrial red dye was added to proceed mitochondrial staining for 30 min, and cells were washed with 37 °C medium for 3 times. Finally, the emission of fluorescent amine at 440-480

nm was detected by laser confocal to quantify $O_2^{-}\cdot$, and the emission of mitochondrial red dye at 590-650 nm was detected to locate mitochondria in cells. As shown in FIG. 3b, both cLAMs and LA can induce mitochondria to produce $O_2^{-}\cdot$, and cLAMs can induce the production of $O_2^{-}\cdot$ more strongly than LA at the same concentration.

Determination of mitochondrial membrane potential: SW480 cells in logarithmic growth active phase were prepared into cell suspension and added into 6-well plate ($5\times10^5$ cells/well). After incubation for 24 h, cells were treated with 1 mM cLAMs and 1 mM LA, and untreated cells were set as blank control group. After incubation for 24 h, the old culture medium was removed, and the cells of each group were collected and centrifuged to obtain pure cells. 1 mL working solution of mitochondrial membrane potential detection kit (JC-1) was added to the collected cells, and then they were mixed thoroughly and incubated for 20 min. Then the mixture was centrifuged at 4 °C to remove working solution, and the cells were washed twice with JC-1 staining buffer (1x) on ice. Finally, 500 μL staining buffer was added to disperse the cells to obtain a cell suspension, and the change of mitochondrial membrane potential was detected by flow cytometry. As shown in FIG. 3c, both cLAMs and LA can induce the decrease of mitochondrial membrane potential, and cLAMs can induce the decrease of mitochondrial membrane potential more strongly than LA at the same concentration.

Determination of cytochrome C release: SW480 cells in logarithmic growth active phase were prepared into cell suspension and added into 6-well plate ($5\times10^5$ cells/well). After incubation for 24 h, cells were treated with 1 mM cLAMs and 1 mM LA, and untreated cells were set as blank control group. After incubation for 24 h, the old culture medium was removed and cells were collected. The collected cells were centrifuged (1200 rpm/min) for 5 min at 4 °C, and the supernatant was removed. The cells were washed with cold PBS twice, and the supernatant was sucked up as much as possible every time. 100 μL of cell lysate containing 1 mM phenylmethylsulfonyl fluoride (PMSF, protease inhibitor) was added to the collected cells, which were placed on ice for 30 min and vortex once every 5 min. Then the mixture was centrifuged at 12000 rpm at 4 °C for 60 min, and the supernatant was quickly collected in another clean centrifuge tube, 20 μL supernatant was taken to analyze the content of cytochrome C in cytoplasm by western blot. The results showed that cLAMs could induce cytochrome C release into cytoplasm more strongly than LA.

Determination of Caspase-3 activity: SW480 cells in logarithmic growth active phase were prepared into cell suspension and added into 6-well plate ($5\times10^5$ cells/well). After incubation for 24 h, cells were treated with 1 mM cLAMs and 1 mM LA, and untreated cells were set as blank control group. After incubation for 24 h, the old culture medium was removed and cells were collected and then centrifuged for 5 min (1200 rpm/min) at 4 °C. The cells were washed with cold PBS twice, and the supernatant was sucked up as much as possible every time. 100 μL of cell lysate containing 1 mM phenylmethylsulfonyl fluoride (PMSF, protease inhibitor) was added to the collected cells, which were placed on ice for 30 min and vortex once every 5 min. Then the mixture was centrifuged at 12000 rpm at 4 °C for 60 min, and the supernatant was quickly collected in another clean centrifuge tube, 20 μL supernatant was taken for detection with Caspase-3 activity detection kit, and the absorbance value at $A_{405}$ nm or $A_{400}$ nm was measured by microplate reader. According to the absorbance ratio of material group cells to blank control group cells, the relative Caspase-3 activity degree was calculated. As shown in FIG. 3d, both cLAMs and LA can stimute the relative activity of Caspase-3, and cLAMs can activate Caspase-3 to induce apoptosis more strongly than LA.

In the above research methods of antitumor mechanism, the lipoic acid micelles were replaced by lipoic acid vesicles and aggregates, respectively, and the same results were obtained.

## EXAMPLE 3

### Antitumor activity of assessment of lipoic acid polymer and LA

Taking lipoic acid micelles (cLAMs) as an example, the antitumor activity of lipoic acid multimer and LA were evaluated.

Human liver cancer cells (HepG2) in the logarithmic growth active phase were selected and inoculated in 96-well plates. After incubation for 24 h, different concentrations of cLAMs and LA were added into the plate respectively, and 5 parallel samples were set for each concentration, and untreated cells were set as blank control group. After incubation for 48 h, the culture medium was removed, and 100 μL fresh medium containing 10% (v/v) MTT was added to each well, and the plates were incubated for another 2 h. Then the culture medium was removed and 150 μL DMSO was added into each well, and the plates were shaked on the oscillator for 2 min. Finally, the absorbance of the solution was measured at 490 nm by using microplate reader. Cell viability was calculated. The results are shown in FIG. 4, cLAMs has better anti-tumor activity than LA, probably because cLAMs enters cells in the form of nanoparticles, and its local concentration in cells is higher than that of LA, so it shows better anti-tumor effect.

In the example, the lipoic acid micelles were replaced by lipoic acid vesicles and aggregates nanoparticles, respectively, and the same results were obtained.

EXAMPLE 4

**Lipoic acid multimer and LA were loaded on liposomes respectively to evaluate the antitumor activity**

Taking lipoic acid micelles (cLAMs) as an example, cLAMs and LA were loaded on liposomes to obtained two kinds of nano-drugs **I** and **II,** and their antitumor activity were evaluated.

1. Preparation of nano-drugs **I** and **II:**

10 mg cLAM and 10 mg LA were dissolved in 20 mL of deionized water respectively, and then 10 mg liposome was added respectively. After ultrasonic oscillation for 2 min, a clear and transparent solution was obtained. The obtained liposome was squeezed through 400 nm polycarbonate membrane for 3 times, and then put into 2 KD dialysis bags respectively, and then lyophilized after dialysis for 48 h to obtain the two nano drugs. cLAMs were physically loaded on liposomes to obtain nano-drug **I;** and LA was physically loaded on liposomes to obtain nano-drug **II.**

2. Evaluation of antitumor effect of nano-drugs **I** and **II:**

Human liver cancer cells (HepG2) in the logarithmic growth active phase were selected and inoculated in 96-well plates. After incubation for 24 h, cells were treated with nano-drugs I and **II,** respectively. Different concentration gradients were set for nano-drugs **I** and **II,** and 5 parallel samples were set for each concentration. Untreated cells were set as blank control group. After incubation for 48 h, the old culture medium was removed, and 100 μL fresh medium containing 10% (v/v) MTT was added to each well. The old culture medium was removed after incubation for 2 h, and 150 μL DMSO was added into each well. After shaking for 2 min in the oscillator, the absorbance of the solution was measured at 490 nm by using microplate reader and the cell viability was calculated. As shown in FIG. 5, nano-drug **I** has a better antitumor activity than nanometer nano-drug **II.**

In the example, the lipoic acid micelles were replaced by lipoic acid vesicles and aggregates nanoparticles, respectively, and the same results were obtained.

**EXAMPLE 5**

**Toxicity evaluation of lipoic acid multimer to normal cells/organisms**

Taking lipoic acid micelles (cLAMs) as an example, the toxicity of lipoic acid multimer to normal cells/organisms was evaluated.

The evaluation of cytotoxicity to normal cells: human renal epithelial cells (293T) and mouse fibroblasts (3T3) in the logarithmic growth phase were selected and inoculated in 96-well plates, respectively. After incubation for 24 h, cLAMs were added into the plates respectively. Different concentration gradients were set for cLAMs, and 5 parallel samples were set for each concentration. Untreated cells were set as blank control group. After incubation for 48 h, the old culture medium was removed, and 100 μL fresh medium containing 10% (v/v) MTT was added to each well. After incubation for another 2 h, the culture media was removed and 150 μL dimethyl sulfoxide was added in each well. After shaking on the oscillator for 2 min, the absorbance of the solution was measured at 490 nm by using microplate reader Varioscan Flash and cell viability was calculated. As shown in FIG. 6, under a certain concentration, cLAMs are non-toxic to 293T and 3T3.

The evaluation of hemolysis: 3 mL fresh blood was collected from the eye socket of normal mice and 12 mL saline was added. The obtained solution was centrifuged at 4°C to collect red blood cells. The obtained red blood cells were washed with saline for 3 times to prepare a 10% (v/v) red blood cell suspension. 50 μL 10% red blood cell suspension was added into 1.5 mL EP tubes, and 7 groups were taken, to which 5 groups were added 950 μL different concentrations of cLAMs (in saline) to make their final concentrations are 30 mg/mL, 20 mg/mL, 10 mg/mL, 5 mg/mL and 1 mg/mL, respectively, and the other two groups were added with 950 μL saline and deionized water as negative control group and positive control group, respectively. After incubation for 2 h in incubator, the obtained samples were centrifugated, and 150 μL supernatant was taken from each group to place in 96-well plate. The absorbance value at 452 nm was detected by microplate reader Varioscan Flash and the hemolysis rate was calculated by formula (1).

$$\text{Hemfigurative ratio }\% = (A_{\text{sample}} - A_{\text{negative}})/(A_{\text{positive}} - A_{\text{negative}})\times 100 \qquad (1)$$

$A_{sample}$ was the absorption value of the material group, $A_{negative}$ was the absorption value of the negative control

group, and $A_{positive}$ was the absorption value of the positive control group. As shown in FIG. 7a, with the increases of concentration of cLAMs, the hemolysis rate increases accordingly. However, until cLAMs reaches the saturated concentration, the hemolysis rate of cLAMs is still lower than 5% (less than 5% is considered normal), indicating that cLAMs does not cause hemolysis.

Coagulation assessment: 50 μL cell suspension (2%) was placed in 6-well plates, and then 950 μL different concentrations of cLAMs (in saline) were added to make the final concentrations were 30 mg/mL, 20 mg/mL, 10 mg/mL, 5 mg/mL and 1 mg/mL, respectively. After incubation at room temperature for 2 h, an inverted fluorescence microscope was used to observe whether there was coagulation. As shown in FIG. 7b, even at a high concentration of cLAMs, there is no obvious coagulation.

Acute toxicity assessment of mice: 15 BALB/c mice (about 20 g each) of 4 weeks old were randomly divided into 3 groups with 5 mice in each group, which were blank control group, saline group and cLAMs group respectively. Through the tail vein, large doses of cLAMs (200 mg/kg) were injected into the cLAMs group, and saline was injected into the saline group at the same time, while the blank control group did not receive any treatment. The weights of the mice were monitored once every 2 days. After two weeks of administration, the blood of mice was collected through eye socket, and blood samples of each group were examined by routine blood test and liver and kidney function test. As shown in FIG. 8, in the cLAMs group, the body weights of mice remains normal after high dose administration, and the blood, liver and kidney functions of the mice are normal, which are consistent with the results of the normal saline group and the blank group.

Through the above experiments, it can be proved that cLAMs almost have no toxic effect on normal cells and body, and also has good biocompatibility.

In the example, the lipoic acid micelles were replaced by lipoic acid vesicles and aggregates nanoparticles, respectively, and the same results were obtained.

**EXAMPLE 6**

**The cytotoxicity evaluation of lipoic acid multimer and traditional chemotherapy drugs to normal cells**

Taking lipoic acid micelles (cLAMs) as an example, the cytotoxicity of gemcitabine hydrochloride (Gem•HCl) and doxorubicin hydrochloride (DOX•HCl) to normal cells cells was compared.

1. Cytotoxicity evaluation of cLAMs, Gem•HCl and DOX•HCl to normal cells:
Human gingival fibroblasts (HGF) in logarithmic growth active phase were selected and inoculated in 96-well plates. After incubation for 24 h, cells were treated with cLAMs, Gem•HCl, and DOX• HCl, respectively. Different concentration gradients were set for each group, and 5 parallel samples were set for each concentration. Untreated cells were set as blank control group. After incubation for 48 h, the old culture medium was removed, and 100 μL fresh medium containing 10% (v/v) MTT was added to each well. After incubation for another 2 h, the culture media was removed and 150 μL dimethyl sulfoxide/well was added. After shaking for 2 min, the absorbance of the solution was measured at 490 nm by using a microplate reader Varioscan Flash and cell viability was calculated. The experimental results are shown in FIG. 9. cLAMs have non-toxicity to normal cells even at higher doses, while Gem•HCl and DOX•HCl can kill normal cells at very low doses.

2. cLAMs, Gem•HCl, and DOX•HCl were physically loaded on liposomes to obtained three kinds of nano-drugs **III, IV** and **V,** and their toxicity to normal cells were evaluated.

Human gingival fibroblasts (HGF) in the logarithmic growth active phase were selected, inoculated into 96-well plates, and cultured for 24 h, and then nano-drug **III,** nano-drug **IV,** and nano-drug **V** were added, respectively. Different concentration gradients were set for nano-drugs **III, IV** and **V,** and 5 parallel samples were set for each concentration. Untreated cells were set as blank control group. After 48 h of culture, the old culture medium was removed, and 100 μL of culture medium containing 10% (v/v) MTT was added to each well. After incubation for 2 h, 150 μL DMSO was added to each well, and the absorbance at 490 nm was measured with a microplate reader for 2 min, and the cell survival rate was calculated. The experimental results are shown in FIG. 10. nano-drug **III** is non-toxic to normal cells at high concentration, while nano-drugs **IV** and **V** have great toxicity to normal cells even at lower concentrations.

In the example, the lipoic acid micelles were replaced by lipoic acid vesicles and aggregates nanoparticles, respectively, and the same results were obtained.

**EXAMPLE 7**

cLAMs and LA were respectively co-loaded with the curcumin, a hydrophobic natural antitumor active substance,

into liposomes to obtain two nano-drugs **VI** and **VII,** and their synergistic antitumor activity was evaluated.

1. The preparation of nano-drugs **VI** and **VII:**

cLAMs (5 mg) and LA (5 mg) were dissolved in 20 mL of deionized water, respectively, and then the liposomes (10 mg) were added. After ultrasonic oscillation for 2 min, a clear and transparent solution was obtained. The obtained liposomes were squeezed to through 400 nm polycarbonate membrane for 3 times to obtain two kinds of liposome containing cLAMs and LA, respectively. Curcumin (5 mg) was dissolved in 200 μL of dimethyl sulfoxide (DMSO), and then the obtained liposomes were added. After shaking in water bath at 60 ° C for 20 min, the solution was put in 2 KD dialysis bags to dialyze for 48 h. After freeze-dried, two kinds of nano-drugs **(VI** and **VII)** were obtained. cLAMs and curcumin were physically loaded on liposomes to obtain nano-drug **VI;** and LA and curcumin were physically loaded on liposomes to obtain nano-drug **VII.**

2. The study of antitumor activity of nano-drugs VI and VII:

Human liver cancer cells (HepG2) in the logarithmic growth active phase were selected and inoculated in 96-well plates. After incubation for 24 h, cells were treated with cLAMs, LA, curcumin, and nano-drugs VI and VII, respectively. Different concentration gradients were set for each group, and 5 parallel samples were set for each concentration. Untreated cells were set as blank control group. After incubation for 48 h, the old culture medium was removed, and fresh medium (100 μL) containing 10% (v/v) MTT was added to each well and the plates were incubated for another 2 h. After incubation, the culture media was removed and 150 μL dimethyl sulfoxide/well was added. After shaking for 2 min, the absorbance of the solution was measured at 490 nm by using a microplate reader Varioscan Flash and cell viability was calculated. The Chou-Talalay combination index formula was used to calculate the combination index (CI) of two drugs. Values of CI < 1 means synergy; CI = 1 means addition; and CI > 1 means antagonism. As shown in FIG. 11, the combination of the two drugs has a better synergistic anti-tumor effect, and the nano-drug **VI** has a better synergistic effect than **VII.** The possible reason is that cLAMs has a better anti-tumor effect than LA, so it has a better synergistic effect with curcumin.

## EXAMPLE 8

### cLAMs were covalently connected to hydrophilic chemotherapy drug cytarabine, and its antitumor activity was evaluated

1. The preparation of nano-drug:

1-(3-dimethylaminopropyl)-3-ethylcarbodiimide hydrochloride (EDCI, 1.2 eq) and N-hydroxysuccinimide (NHS, 1.2 eq) were added to a proper amount of cLAMs aqueous solution. After reaction for 2 h, cytarabine (1 eq) was added. After 24 h of continuous reaction, the reaction solution was put into a 2 KD dialysis bag for dialysis for 48 h, and the dialysate was lyophilized to obtain nano-drugs.

2. The study of antitumor activity of nano-drug **VIII:**

SW480 cells in the logarithmic growth active phase were selected and inoculated in 96-well plates. After incubation for 24 h, cells were treated with cytarabine, cLAMs, LA, [cytarabine+cLAMs], [cytarabine+LA] and nano-drug **VIII,** respectively. Different concentration gradients were set for each group, and 5 parallel samples were set for each concentration. Untreated cells were set as blank control group. After incubation for 48 h, the culture medium was removed, and fresh medium (100 μL) containing 10% (v/v) MTT was added to each well and the plates were incubated for another 2 h. After incubation, the culture media was removed and 150 μL dimethyl sulfoxide/well was added. After shaking for 2 min, the absorbance of the solution was measured at 490 nm by using a microplate reader Varioscan Flash and cell viability was calculated. CI values of [cytarabine+cLAMs], [cytarabine+LA] and nano-drug **VIII** were calculated. As shown in FIG. 12, the combination of the two drugs can increase the toxicity of the drug, and [cytarabine+cLAMs] and [cytarabine+LA] have certain synergistic effects. However, the nano-drug prepared in this example has the best synergistic anti-tumor effect, mainly because the two drugs in the nano-drug can enter the cell at a predetermined ratio, thus showing a better synergistic effect.

## EXAMPLE 9

Lipoic acid was covalently connected with chemotherapeutic drug hydroxyurea to form small molecule monomers, which were then prepared into nanoparticles by coprecipitation method, and then nano-drugs were obtained by disulfide polymerization of lipoic acid, and synergistic anti-tumor effect was evaluated.

1. Preparation of small molecule

Lipoic acid (200 mg), *N*-hydroxysuccinimide (HOSU, 138 mg), dicyclohexylcarbodiimide (DCC, 247 mg) were dissolved in tetrahydrofuran (7 mL). The obtained solution was stirred at room temperature for 6 h under the protection of nitrogen, and then the precipitates were removed after filtration. The filtrate was concentrated to obtain light yellow powder, which was directly dissolved in 8 mL of dry DMF together with 76 mg of hydroxyurea, and 0.25 mL of triethylamine was added at the same time. Under nitrogen protection, the solution was stirred at room temperature for 48 h, and DMF was removed under reduced pressure. The remaining solid was purified by column chromatography ($CH_2Cl_2$: MeOH = 10: 1) to obtain the target small molecule.

2. Preparation of nano-drug **IX:**

20 mg of the small molecular monomer was weighed and dissolved in 200 μL of dimethyl sulfoxide (DMSO), and then dropped into 20 mL of deionized water under ultrasonic condition until a clear and transparent system was formed. The self-crosslinking of lipoic acid disulfide bonds was initiated by ultraviolet light at 365 nm, and the reaction lasted for 2.5 h. After dialysis for 48 h, the dialysate was lyophilized to obtain a nano-drug **IX.**

3. The study of antitumor activity of nano-drug **IX:**

HepG2 cells in the logarithmic growth active phase were selected and inoculated in 96-well plates. After incubation for 24 h, cells were treated with LA, hydroxyurea, [LA+hydroxyurea] and nano-drug **IX** prepared in the example, respectively. Different concentration gradients were set for each group, and 5 parallel samples were set for each concentration. Untreated cells were set as blank control group. After incubation for 48 h, the old culture medium was removed, and fresh medium (100 μL) containing 10% (v/v) MTT was added to each well and the plates were incubated for another 2 h. After incubation, the culture media was removed and 150 μL dimethyl sulfoxide/well was added. After shaking for 2 min, the absorbance of the solution was measured at 490 nm by using a microplate reader Varioscan Flash and cell viability was calculated. CI values of [LA+hydroxyurea] and nano-drug **IX** were calculated. As shown in FIG. 13, both [LA+hydroxyurea] and nano-drug **IX** have good synergistic antitumor effect, but the nano-drug **IX** has a better synergistic effect than [LA+hydroxyurea], mainly because the two drugs in nano-drug **IX** can enter the cell at a predetermined ratio, thus resulting in a better synergistic effect.

**EXAMPLE 10**

Lipoic acid could be directly mixed with natural antitumor active substances lycopene and anthocyanin to form nanoparticles. Lipoic acid was further polymerized to form nano-drugs **X,** and its antitumor activity was evaluated.

1. Preparation of nano-drug **X:**

8.26 mg of LA, 3 mg of lycopene and 1 mg of anthocyanin were weighed and dissolved in 200 μL of DMSO, and then dropped into 20 mL of deionized water under ultrasonic condition until a clear and transparent system was formed. After 365 nm ultraviolet light irradiation, lipoic acid disulfide bonds were self-crosslinked and reacted for 2.5 h. After dialysis for 48 h, the dialysate was lyophilized to obtain nano-drug **X.**

**2.** The study of antitumor activity of nano-drug X:

HepG2 cells in the logarithmic growth active phase were selected and inoculated in 96-well plates. After incubation for 24 h, cells were treated with LA, lycopene, anthocyanin, [lipoic acid+lycopene+anthocyanin] and nano-drug **X** prepared in the example, respectively. Different concentration gradients were set for each group, and 5 parallel samples were set for each concentration. Untreated cells were set as blank control group. After incubation for 48 h, the old culture medium was removed, and fresh medium (100 μL) containing 10% (v/v) MTT was added to each well and the plates were incubated for another 2 h. After incubation, the culture media was removed and 150 μL dimethyl sulfoxide/well was added. After shaking for 2 min, the absorbance of the solution was measured at 490 nm by using a microplate reader Varioscan Flash and cell viability was calculated. CI values of [lipoic acid+lycopene+anthocyanin] and nano-drug **X** were calculated. As shown in FIG. 14, both [lipoic acid+lycopene+anthocyanin] and nano-drug **X** have good synergistic antitumor effect, but the nano-drug **X** has a better synergistic effect than [lipoic acid+lycopene+anthocyanin], mainly

because three drugs in nano-drug **X** can enter the cell at a predetermined ratio, thus result in a better synergistic effect.

**EXAMPLE 11**

Cross-lipoic acid vesicles (cLAVs) were physically loading hydrophilic chemotherapeutic drug hydroxyurea to obtain nano-drug **XI,** and its synergistic antitumor effect was evaluated.

1. Preparation of nano-drug **XI:**

The pH value of the cLAVs solution containing supersaturated hydroxyurea was adjusted to alkaline. After swelling and shaking in water bath at 37 °C overnight, the solution was adjusted to neutral and put in 2 KD dialysis bags to dialyze for 48 h. The dialysis solution was freezy-dried to obtain the nano-drug **XI.**

**2.** The study of antitumor activity of nano-drug **XI:**

SW480 cells in the logarithmic growth active phase were selected and inoculated in 96-well plates. After incubation for 24 h, cells were treated with hydroxyurea, LA, cLAVs, [hydroxyurea+LA], [hydroxyurea+cLAVs] and nano-drug **XI,** respectively. Different concentration gradients were set for each group, and 5 parallel samples were set for each concentration. Untreated cells were set as blank control group. After incubation for 48 h, the old culture medium was removed, and fresh medium (100 μL) containing 10% (v/v) MTT was added to each well and the plates were incubated for another 2 h. After incubation, the culture media was removed and 150 μL dimethyl sulfoxide/well was added. After shaking for 2 min, the absorbance of the solution was measured at 490 nm by using a microplate reader Varioscan Flash and cell viability was calculated. CI values of [hydroxyurea+LA], [hydroxyurea+cLAVs] and nano-drug **XI** were calculated. As shown in FIG. 15, both [hydroxyurea+LA], [hydroxyurea+cLAVs] and nano-drug **XI** have good synergistic antitumor effect, but the nano-drug **XI** has a better synergistic effect than [hydroxyurea+LA], [hydroxyurea+cLAVs], mainly because three drugs in nano-drug **XI** can enter the cell at a predetermined ratio, thus resulting in a better synergistic effect.

**EXAMPLE 12**

Taking nano-drug XI as an example, its synergistic antitumor mechanism was evaluated.

Determination of superoxide anion ($O_2^{-\cdot}$) in mitochondria: SW480 cells in logarithmic growth active phase were prepared into cell suspension and added into 6-well plate ($5\times10^5$ cells/well). After incubation for 24 h, cells were treated with 1 mM hydroxyurea, 1 mM cLAVs and 1 mM nano-drug XI, respectively, and untreated cells were set as blank control group. After incubation for 4 h, the old culture medium was removed and 1 mL of fluorescent amine dye (50 μM) was added. After incubation for 2 h, cysteine was added to make the final concentration to be 200 μM. After incubation for 0.5 h, culture medium was removed and cells were washed with Krebs buffer for 3 times, then mitochondrial red dye was added to proceed mitochondrial staining for 30 min, and cells were washed with 37 °C medium for 3 times. Finally, the emission of fluorescent amine at 440-480 nm was detected by laser confocal to quantify $O_2^{-\cdot}$, and the emission of mitochondrial red dye at 590-650 nm was detected to locate mitochondria in cells. As shown in FIG. 16a, hydroxyurea, cLAVs and nanodurg XI can all induce mitochondria to produce $O_2^{-\cdot}$, and the nano-drug of this example can induce the production of $O_2^{-\cdot}$ more strongly at the same concentration.

Determination of mitochondrial membrane potential: SW480 cells in logarithmic growth active phase were prepared into cell suspension and added into 6-well plate ($5\times10^5$ cells/well). After incubation for 24 h, cells were treated with 1 mM hydroxyurea, 1 mM cLAVs and 1 **mM** nano-drug **XI,** respectively, and ntreated cells were set as blank control group. After incubation for 24 h, the old culture medium was removed, and the cells of each group were collected and centrifuged to obtain pure cells. 1 mL working solution of mitochondrial membrane potential detection kit (JC-1) was added to the collected cells, and then they were mixed thoroughly and incubated for 20 min. Then the mixture was centrifuged at 4 °C to remove working solution, and the cells were washed twice with JC-1 staining buffer (1x) on ice. Finally, 500 μL staining buffer was added to disperse the cells to obtain a cell suspension, and the change of mitochondrial membrane potential was detected by flow cytometry. As shown in FIG. 16b, hydroxyurea, cLAVs and nano-drug **XI** can all induce the decrease of mitochondrial membrane potential, and the nano-drug of this example can induce the decrease of mitochondrial membrane potential more strongly at the same concentration.

Determination of cytochrome C release: SW480 cells in logarithmic growth active phase were prepared into cell suspension and added into 6-well plate ($5\times10^5$ cells/well). After incubation for 24 h, cells were treated with 1 mM hydroxyurea, 1 mM cLAVs and 1 mM nano-drug **XI,** respectively, and untreated cells were set as blank control group. After incubation for 24 h, the old culture medium was removed and cells were collected. The collected cells were centrifuged (1200 rpm/min) for 5 min at 4 °C, and the supernatant was removed. The cells were washed with cold PBS

twice, and the supernatant was sucked up as much as possible every time. 100 μL of cell lysate containing 1 mM phenylmethylsulfonyl fluoride (PMSF, protease inhibitor) was added to the collected cells, which were placed on ice for 30 min and vortex once every 5 min. Then the mixture was centrifuged at 12000 rpm at 4 °C for 60 min, and the supernatant was quickly collected in another clean centrifuge tube, 20 μL supernatant was taken to analyze the content of cytochrome C in cytoplasm by western blot. The results showed that the nano-drug of this example can induce cytochrome C release into cytoplasm more strongly than hydroxyurea and cLAVs.

Determination of Caspase-3 activity: SW480 cells in logarithmic growth active phase were prepared into cell suspension and added into 6-well plate ($5\times10^5$ cells/well). After incubation for 24 h, cells were treated with 1 mM hydroxyurea, 1 mM cLAVs and 1 mM nano-drug **XI,** respectively, and untreated cells were set as blank control group. After incubation for 24 h, the old culture medium was removed and cells were collected and then centrifuged for 5 min (1200 rpm/min) at 4 °C. The cells were washed with cold PBS twice, and the supernatant was sucked up as much as possible every time. 100 μL of cell lysate containing 1 mM phenylmethylsulfonyl fluoride (PMSF, protease inhibitor) was added to the collected cells, which were placed on ice for 30 min and vortex once every 5 min. Then the mixture was centrifuged at 12000 rpm at 4 °C for 60min, and the supernatant was quickly collected in another clean centrifuge tube, 20 μLsupernatant was taken for detection with Caspase-3 activity detection kit, and the absorbance value at $A_{405}$ nm or $A_{400}$ nm was measured by microplate reader. According to the absorbance ratio of material group cells to blank control group cells, the relative Caspase-3 activity degree was calculated. As shown in FIG. 16c, hydroxyurea, cLAVs and nano-drug **XI** can stimulate the relative activity of Caspase-3, and nano-drug **XI** can activate Caspase-3 to induce apoptosis more strongly than hydroxyurea and cLAVs.

The results show that hydroxyurea and cLAVs can induce mitochondria to produce ROS, and induce the decrease of mitochondrial membrane potential, promote the release of cytochrome C from the mitochondria to the cytoplasm, and then regulate the Caspase-3-dependent apoptosis pathway.

## EXAMPLE 13

Taking nano-drug **XI** as an example, its synergistic antitumor mechanism *in vivo* was evaluated.

Subcutaneous SW480 xenograft model was established in BALB/c nude mice (about 20 g each) of 4 weeks old. When the tumor reached to about 100 $mm^3$, nude mice were randomly divided into 7 groups and 5 mice in each group, namely the lipoic acid group (LA, 10 mg/kg), the lipoic acid vesicle group (cLAVs, 10 mg/kg), the hydroxyurea group (Hydrea, 6 mg/kg), and the hydroxyl urea+lipoic acid group (Hydrea +LA, Hydrea: 6 mg/kg. LA: 10 mg/kg), hydroxyurea+cLAVs group (Hydrea+cLAVs, Hydrea: 6 mg/kg, cLAVs: 10 mg/kg) and the nano-drug **XI** group (Hydrea: cLAVs =1:1.67, Hydrea: 6 mg/kg, cLAVs: 10 mg/kg), while saline group was set as the blank control. The 7 groups of nude mice were given the drug once every 3 days for 8 times in total, during which tumor volume and body weight of the mice were recorded. As shown in FIG. 17, compared with the LA group, there was an obvious tumor inhibition effect in cLAVs group, indicating that cLAVs can effectively promote the enrichment of drugs in the tumor tissue, thus improving the efficacy of lipoic acid. Hydroxyurea showed a good tumor inhibition effect, mainly because it is a small-molecule chemotherapy drug, which can inhibit tumor growth at a lower concentration. Both the [hydroxyurea+LA] group and [hydroxyurea+cLAVs] group have excellent tumor inhibition effect, mainly because the combination of hydroxyurea with LA and cLAVs, while the latter has a better antitumor effect, mainly because cLAVs has higher tumor enrichment than LA. The nano-drug **XI** have the best tumor inhibition effect, mainly because the nanoparticles can be passively targeted to the tumor tissue, mainly because hydroxyurea and cLAVs can enter the cell in a predetermined ratio.

## EXAMPLE 14

In Example 1, the mixture of (*R*)-(+)-lipoic acid and (*S*)-(-)-lipoic acid were used as raw material instead of (R)-(+)- lipoic acid, and a series of lipoic acid polymers containing R-type chiral structure were successfully prepared. Meanwhile, the anti-tumor effects of the obtained lipoic acid polymer and the anti-tumor nano-drugs obtained by combining the obtained lipoic acid polymer with traditional drugs were evaluated with reference to the methods of Examples 2-13. The results show that the hybrid lipoic acid multimers obtained in this example, compared with the lipoic acid multimers completely composed of (*R*)-(+)-lipoic acid in example 1, have basically the same beneficial effect. That is, both of them can significantly improve the antitumor activity compared with the lipoic acid monomer. Both of them, combined with several traditional drugs, can achieve a synergistic antitumor effect of "1+1 > 2".

## EXAMPLE 15

In example 1, 0.1 equivalent of dithiothreitol (DTT) was used instead of UV to initiate ring-opening polymerization of disulfide bonds. After reaction for 12 h and dialysis for 48 h, a series of lipoic acid multimers doped with small amounts of DTT were successfully obtained, which were micelles, vesicles and nanoparticles. Meanwhile, the anti-tumor effect

of the obtained lipoic acid polymer and the anti-tumor nano-drug obtained by combining the lipoic acid polymer with traditional drugs was evaluated by referring to the methods of Examples 2-13. The results show that the hybrid lipoic acid multimers obtained in this example, compared with the lipoic acid multimers completely composed of (*R*)-(+)-lipoic acid in Example 1, have basically the same beneficial effect. That is, both of them can significantly improve the antitumor activity compared with the lipoic acid monomer. Both of them, combined with several traditional drugs, can achieve a synergistic antitumor effect of " 1+1 > 2".

The above is only a preferred embodiment of the disclosure, which is only illustrative, not restrictive; Those skilled in the art will understand that many changes, modifications and even equivalent changes can be made within the spirit and scope of the invention as defined by the claims, but all of them will fall into the protection scope of the disclosure.

## Claims

1. An antitumor nano-drug, wherein the antitumor active ingredient mainly exists in the form of lipoic acid multimer.

2. The nano-drug according to claim 1, wherein the lipoic acid multimer exists in the form of at least one of chain polymers, micelle, vesicles and aggregates.

3. The nano-drug according to claim 1, wherein the lipoic acid multimer can be used in combination with other antitumor active substances.

4. The nano-drug according to claim 1, wherein the lipoic acid multimer can cooperate with other antitumor active substances to achieve the antitumor effect of "1+1 > 2".

5. The nano-drug according to claim 4, wherein the other antitumor active substances can be any substance capable of producing a synergistic antitumor effect with a lipoic acid multimer.

6. The nano-drug according to claim 4, wherein the other antitumor active substances are at least one of traditional chemotherapeutic drugs, such as camptothecin, hydroxyurea, pixantrone, doxorubicin hydrochloride, gemcitabine hydrochloride, cytarabine, or natural antitumor active substances, such as anthocyanins, resveratrol, curcumin, licopersicin, tea polyphenol, and astaxanthin.

7. The nano-drug according to claim 1, wherein the lipoic acid multimer is loaded on the nano-drug carrier.

8. The nano-drug according to claim 7, wherein the nano-drug carrier is specifically liposome, dendrimer, polymer micelle, vesicle or lipoic acid multimer.

9. The nano-drug according to claim 1, wherein the lipoic acid multimer can also act as a nano-drug carrier.

10. A use of lipoic acid multimer in preparing the antitumor nano-drug

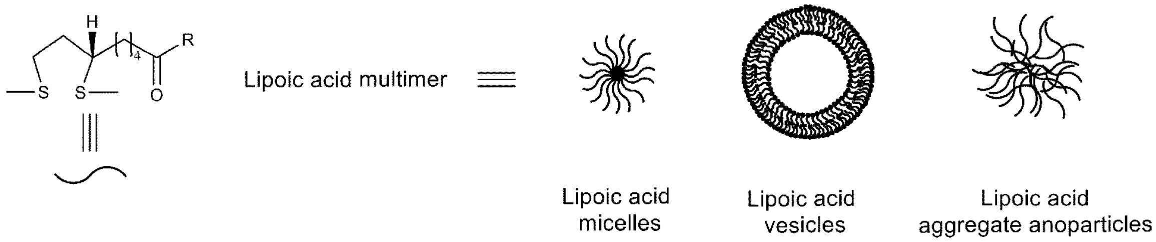


FIG. 1

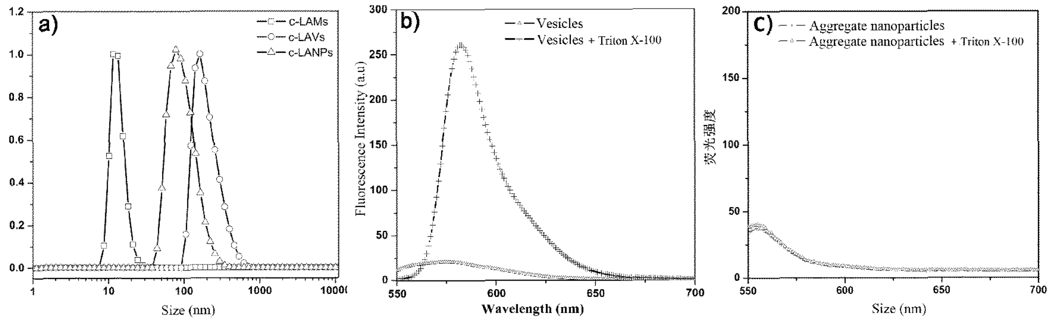


FIG. 2

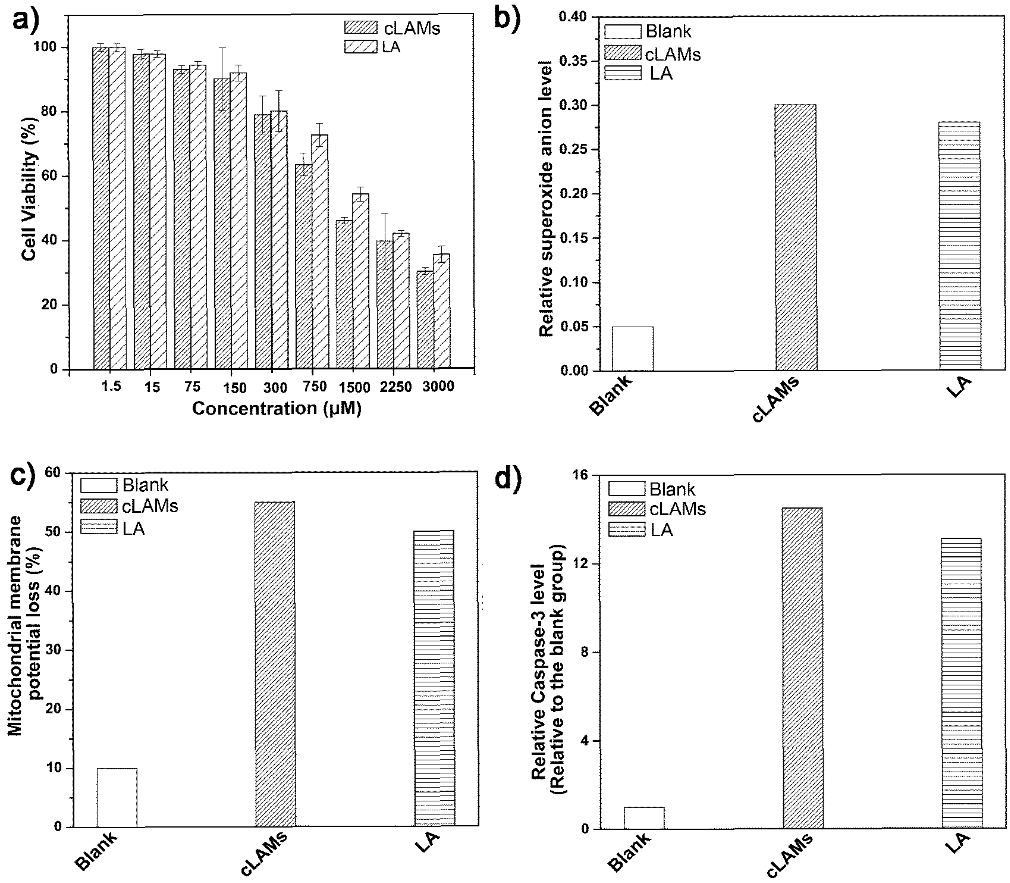


FIG. 3

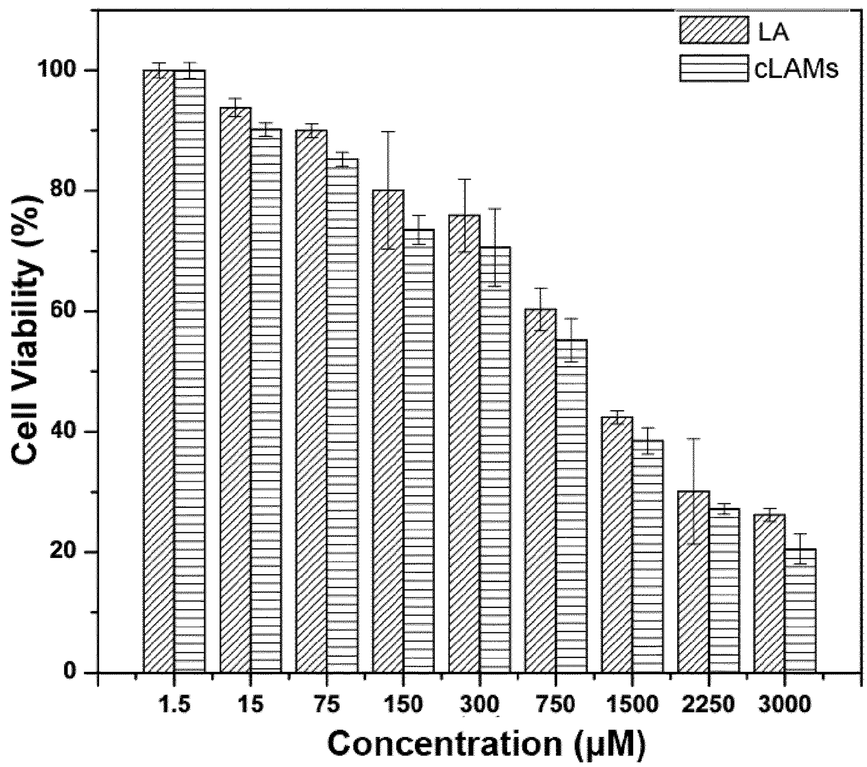
LA
cLAMs
Cell Viability (%)
0
20
40
60
80
100
1.5
15
75
150
300
750
1500
2250
3000
Concentration (μM)

FIG. 4

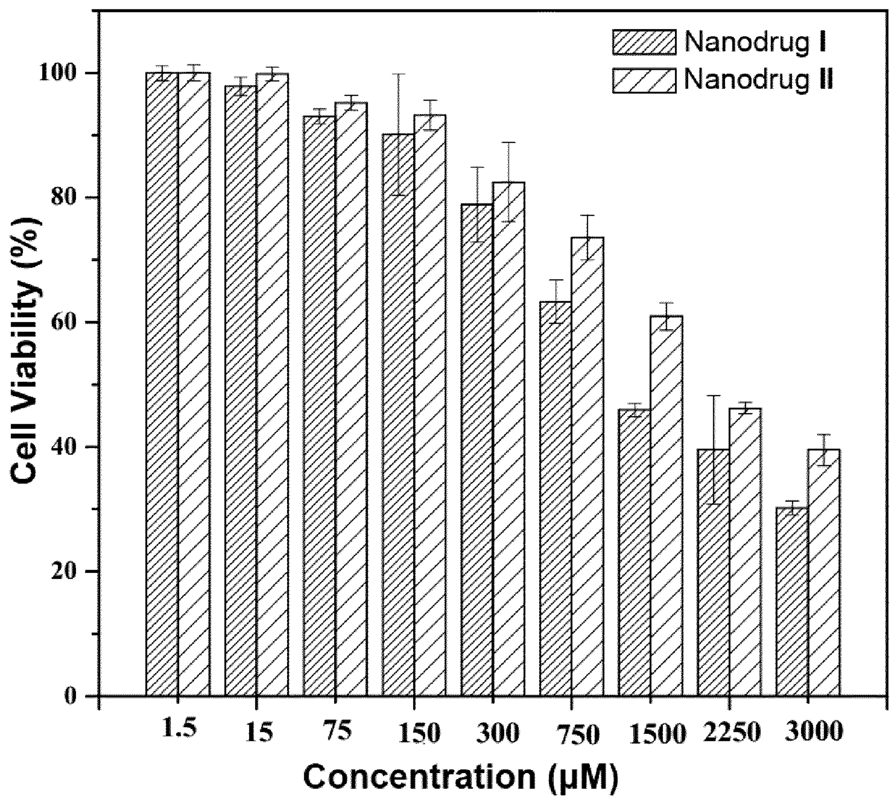
Nanodrug I
Nanodrug II
Cell Viability (%)
0
20
40
60
80
100
1.5
15
75
150
300
750
1500
2250
3000
Concentration (μM)

FIG. 5

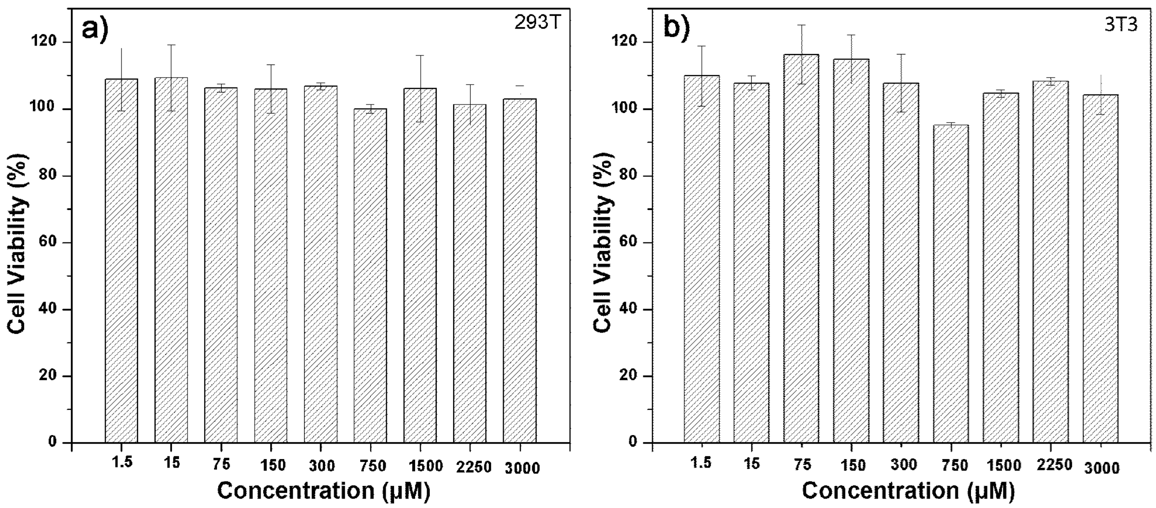
a)
293T
Cell Viability (%)
Concentration (μM)
1.5
15
75
150
300
750
1500
2250
3000
b)
3T3
Cell Viability (%)
Concentration (μM)

FIG. 6

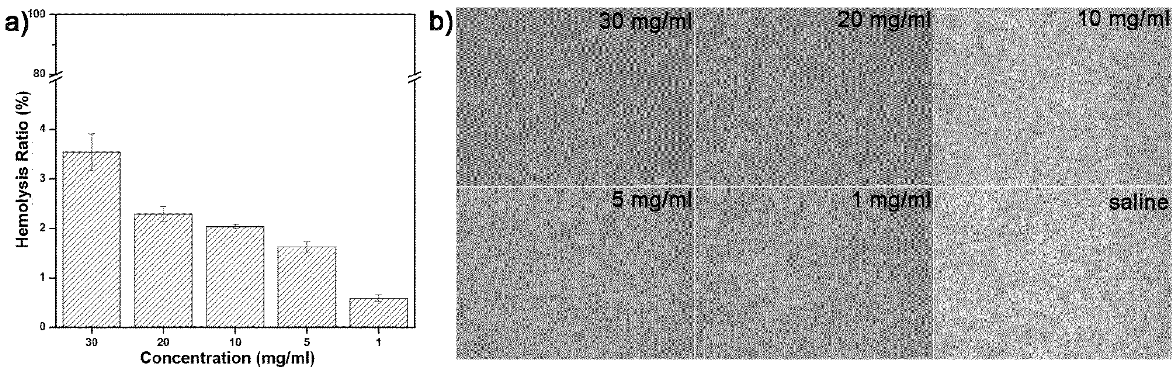
a)
Hemolysis Ratio (%)
Concentration (mg/ml)
30
20
10
5
1
b)
30 mg/ml
20 mg/ml
10 mg/ml
5 mg/ml
1 mg/ml
saline

FIG. 7

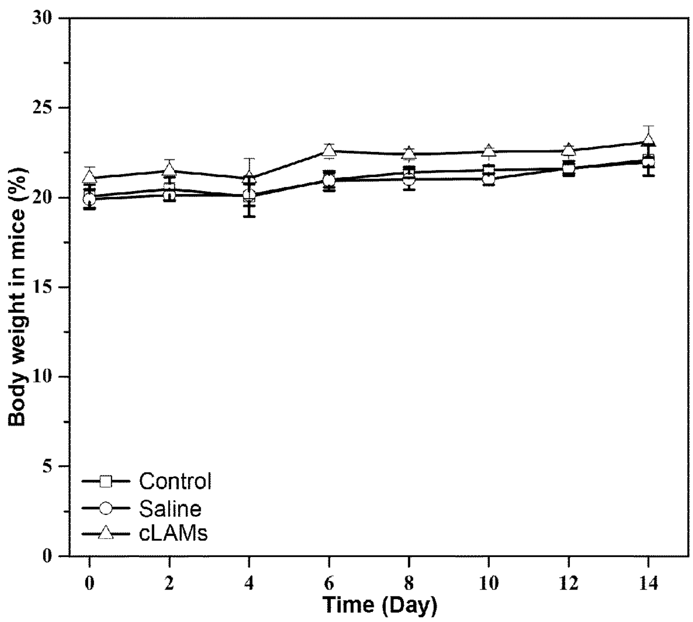
Body weight in mice (%)
Time (Day)
Control
Saline
cLAMs

FIG. 8

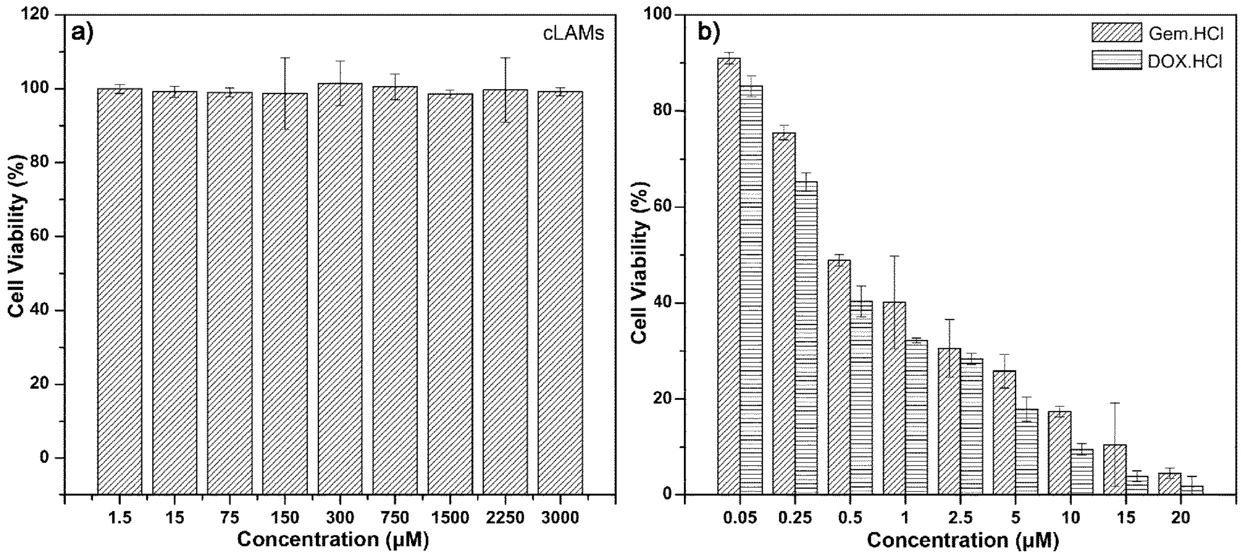


FIG. 9

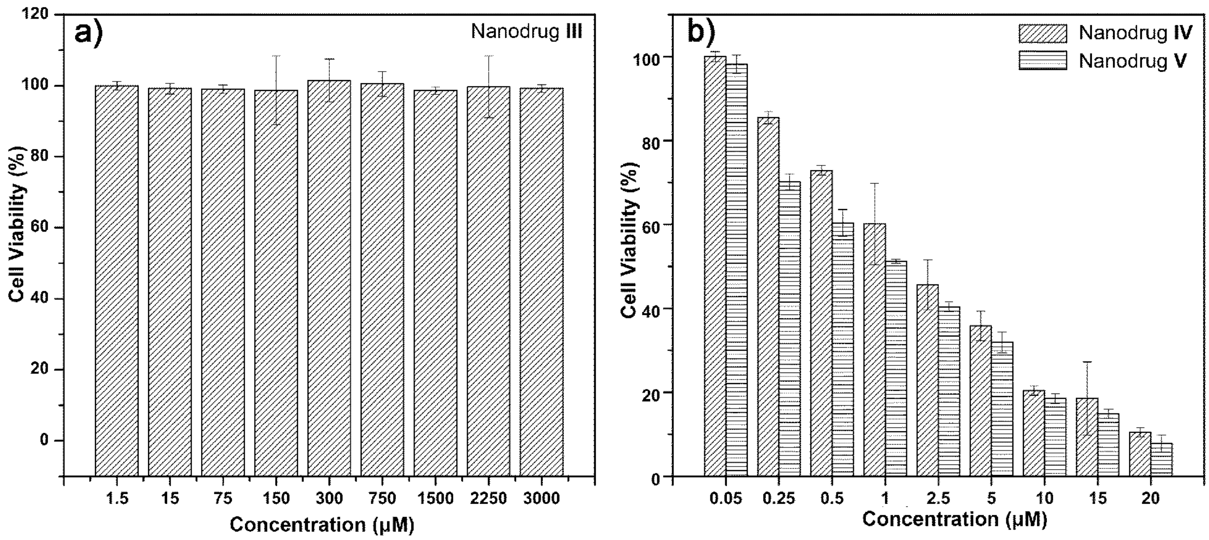


FIG. 10

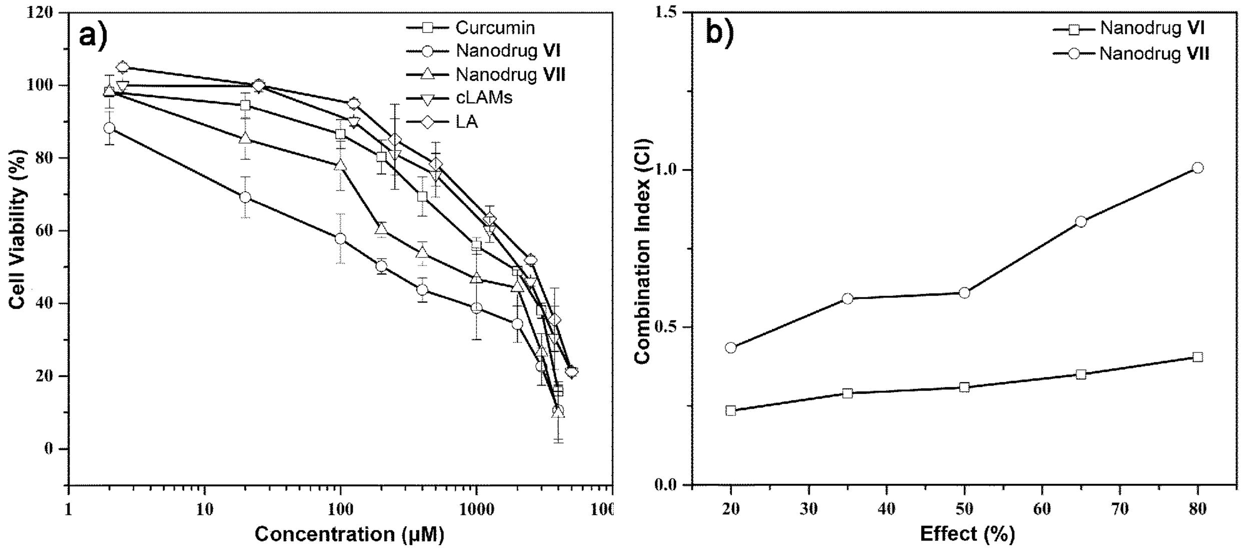
a)
Curcumin
Nanodrug VI
Nanodrug VII
cLAMs
LA
Cell Viability (%)
Concentration (μM)
b)
Nanodrug VI
Nanodrug VII
Combination Index (CI)
Effect (%)

FIG. 11

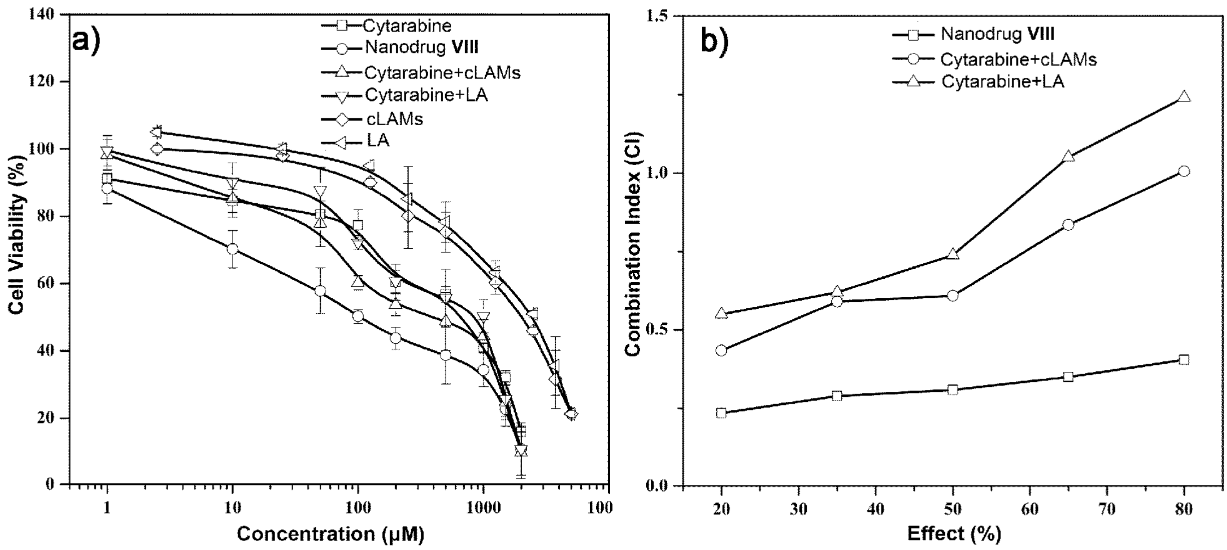
a)
Cytarabine
Nanodrug VIII
Cytarabine+cLAMs
Cytarabine+LA
cLAMs
LA
Cell Viability (%)
Concentration (μM)
b)
Nanodrug VIII
Cytarabine+cLAMs
Cytarabine+LA
Combination Index (CI)
Effect (%)

FIG. 12

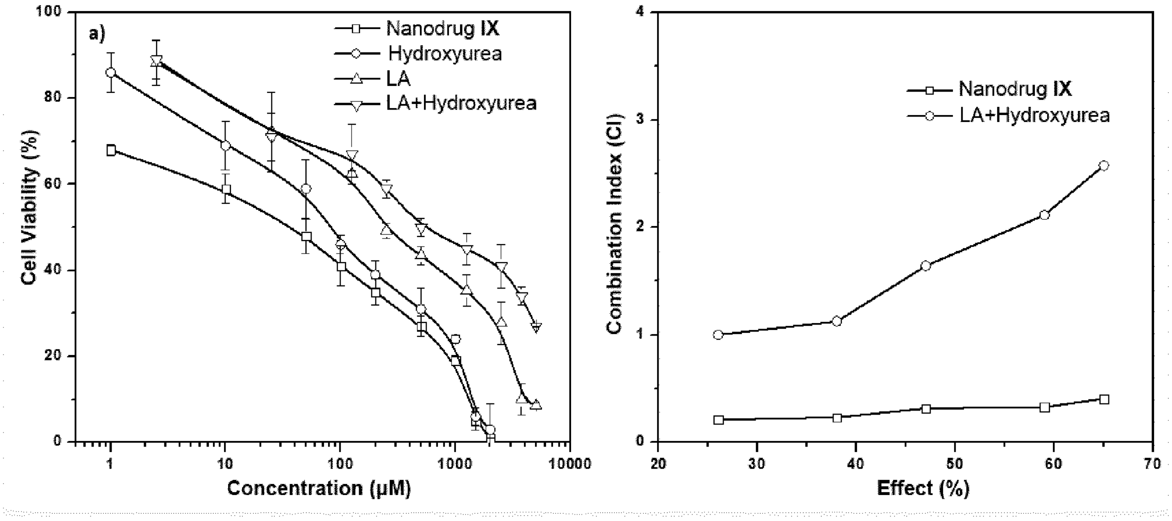


FIG.

FIG.13

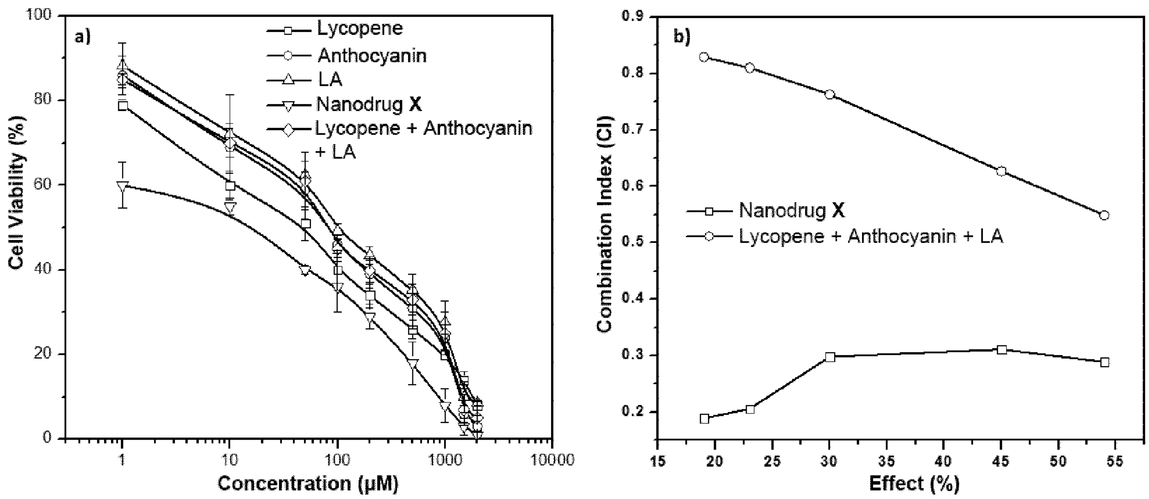


FIG. 14

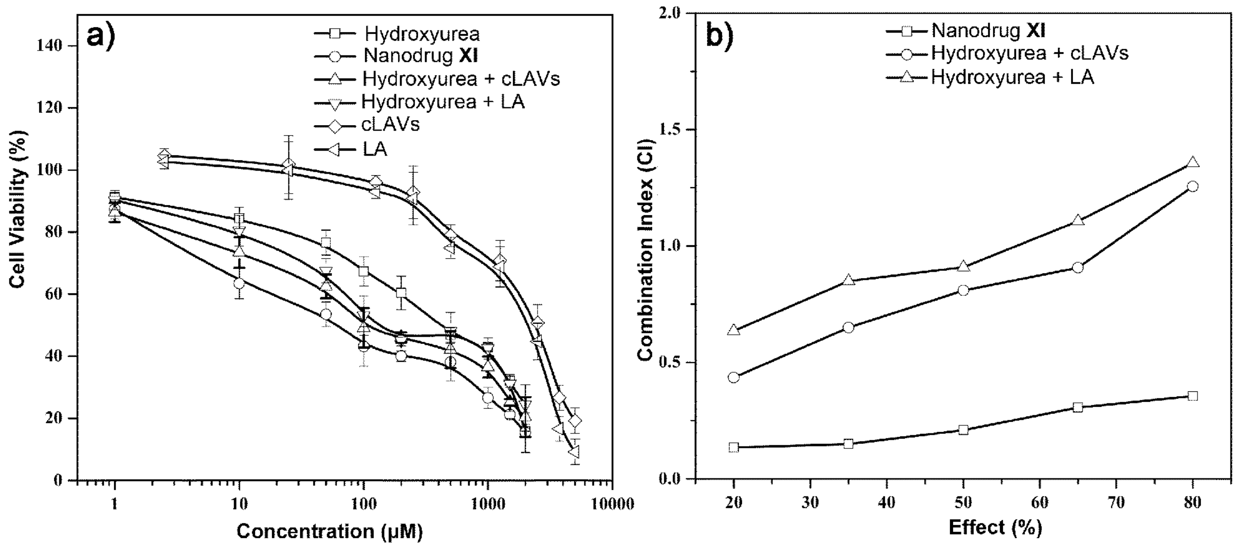
a)
Hydroxyurea
Nanodrug XI
Hydroxyurea + cLAVs
Hydroxyurea + LA
cLAVs
LA
Cell Viability (%)
Concentration (μM)
b)
Nanodrug XI
Hydroxyurea + cLAVs
Hydroxyurea + LA
Combination Index (CI)
Effect (%)

FIG. 15

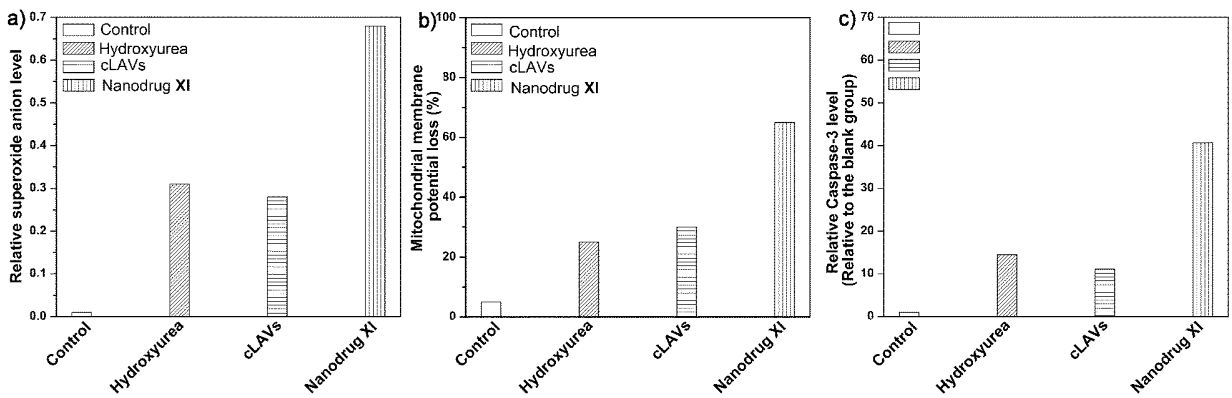
a)
Control
Hydroxyurea
cLAVs
Nanodrug XI
Relative superoxide anion level
b)
Mitochondrial membrane potential loss (%)
c)
Relative Caspase-3 level (Relative to the blank group)

FIG. 16

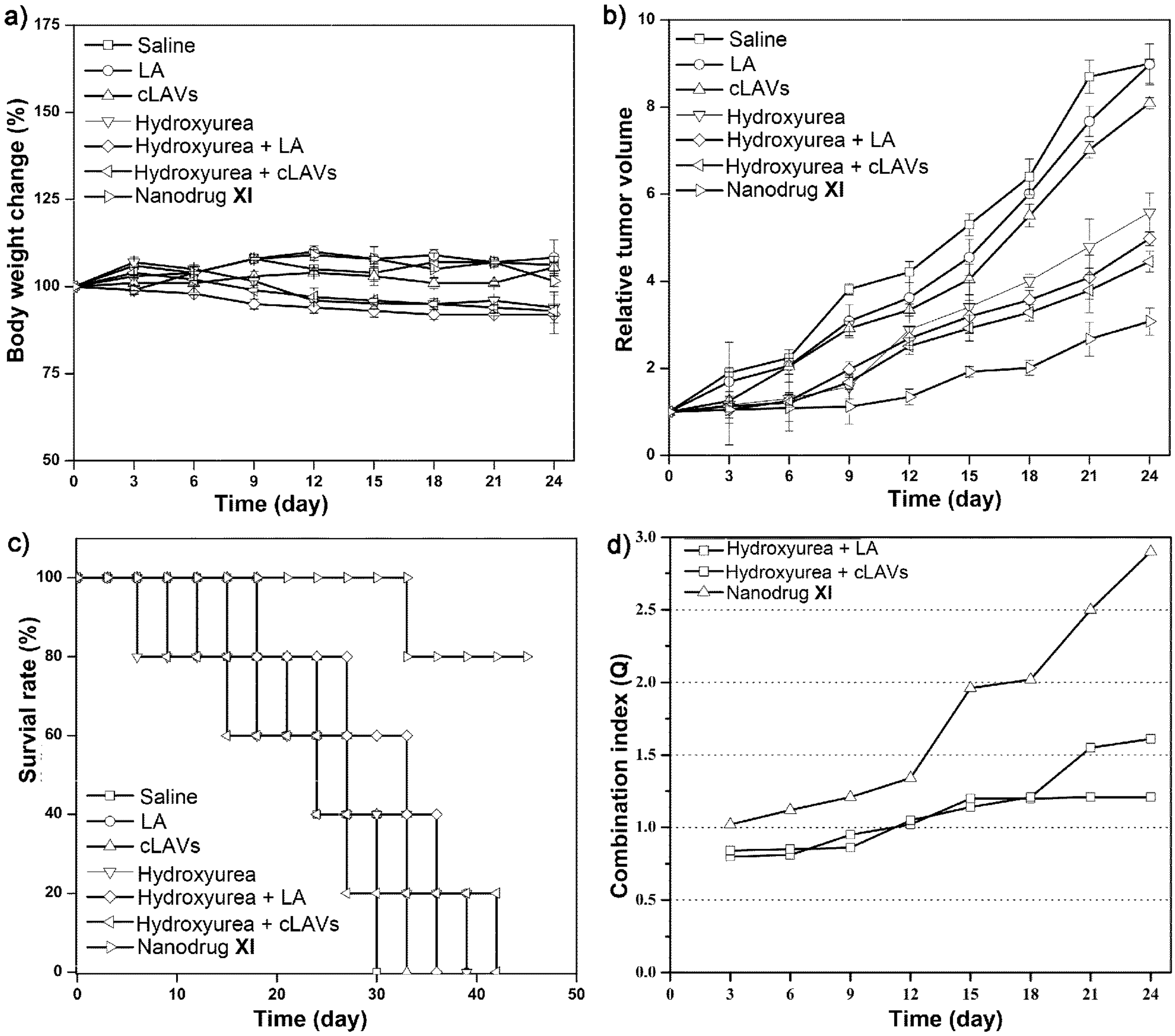
a)
Saline
LA
cLAVs
Hydroxyurea
Hydroxyurea + LA
Hydroxyurea + cLAVs
Nanodrug **XI**
Body weight change (%)
Time (day)
b)
Saline
LA
cLAVs
Hydroxyurea
Hydroxyurea + LA
Hydroxyurea + cLAVs
Nanodrug **XI**
Relative tumor volume
Time (day)
c)
Saline
LA
cLAVs
Hydroxyurea
Hydroxyurea + LA
Hydroxyurea + cLAVs
Nanodrug **XI**
Survial rate (%)
Time (day)
d)
Hydroxyurea + LA
Hydroxyurea + cLAVs
Nanodrug **XI**
Combination index (Q)
Time (day)

FIG. 17

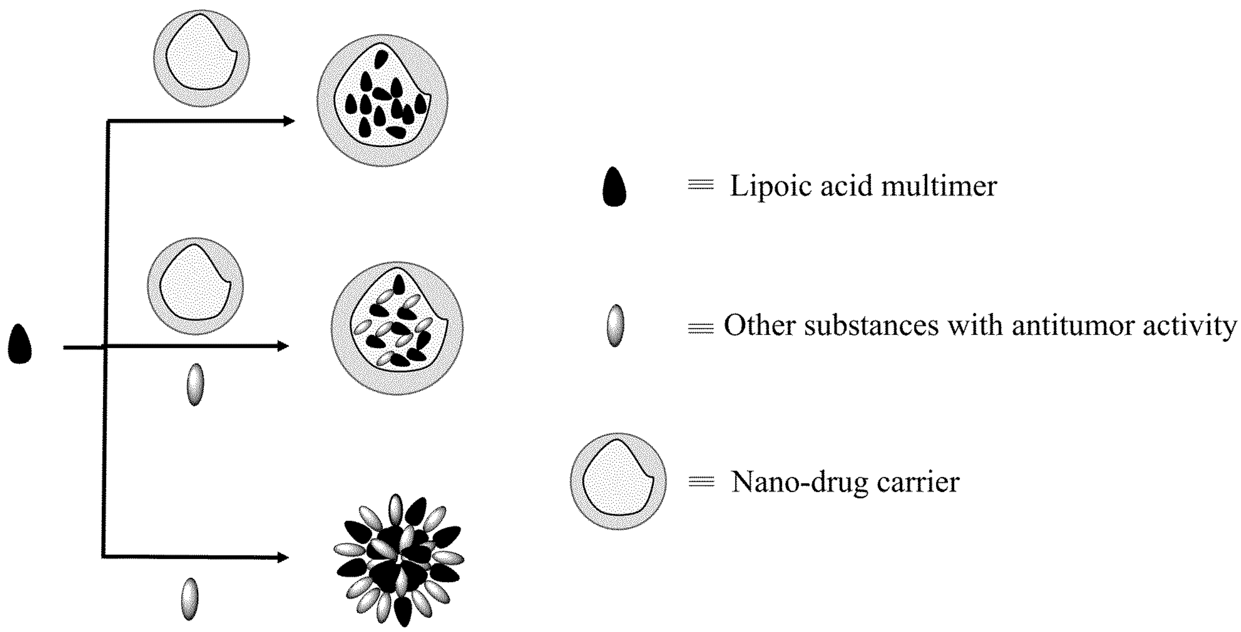
≡ Lipoic acid multimer
≡ Other substances with antitumor activity
≡ Nano-drug carrier

FIG. 18

**INTERNATIONAL SEARCH REPORT**

International application No.
**PCT/CN2019/083390**

**A. CLASSIFICATION OF SUBJECT MATTER**

A61K 31/385(2006.01)i; A61K 31/795(2006.01)i; A61K 47/34(2017.01)i; A61K 9/127(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K A61P C08G

Documentation searched other than minimum documentation to the extent that such documents are included in the fields searched

Electronic data base consulted during the international search (name of data base and, where practicable, search terms used)

CNABS; CPRSABS; CNTXT; TWTXT; CNKI; 读秀; DUXIU; DWPI; EPODOC; CA; NCBI: 硫辛酸, 手性, 多聚体, 聚合物, 胶束, 囊泡, 聚集体, 喜树碱, 吉西他滨, 花青素, 纳米, 抗肿瘤, alpha lipoic acid, trioctic acid, chirality, R, multimer, polymer, micella, micelle, vesica, camptothecin, gemcitabine, gem, anthocyanin, nano, antitumor, antineoplastic

**C. DOCUMENTS CONSIDERED TO BE RELEVANT**

| Category* | Citation of document, with indication, where appropriate, of the relevant passages | Relevant to claim No. |
|---|---|---|
| X | CN 105999299 A (SICHUAN UNIVERSITY) 12 October 2016 (2016-10-12) embodiment 1, and description, paragraphs [0037] and [0040] | 1-10 |
| X | CN 104629054 A (INSTITUTE OF CHEMISTRY CHINESE ACADEMY OF SCIENCES) 20 May 2015 (2015-05-20) embodiment 1, description, paragraph [0004], and claim 10 | 1-10 |
| PX | CN 108553458 A (SICHUAN UNIVERSITY) 21 September 2018 (2018-09-21) claims 1-10 | 1-10 |
| A | CN 106083811 A (SUZHOU FUSHILAI PHARMACEUTICAL CO., LTD.) 09 November 2016 (2016-11-09) description, paragraphs [0002]-[0004] | 1-10 |
| A | CN 101721369 A (GUANGDONG PHARMACEUTICAL UNIVERSITY) 09 June 2010 (2010-06-09) claims 1-10 | 1-10 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 July 2019** | **22 July 2019** |

| Name and mailing address of the ISA/CN | Authorized officer |
|---|---|
| **State Intellectual Property Office of the P. R. China (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2019/083390**

| Patent document cited in search report | | | Publication date (day/month/year) | Patent family member(s) | | | Publication date (day/month/year) |
|---|---|---|---|---|---|---|---|
| CN | 105999299 | A | 12 October 2016 | None | | | |
| CN | 104629054 | A | 20 May 2015 | CN | 104629054 | B | 25 January 2017 |
| CN | 108553458 | A | 21 September 2018 | None | | | |
| CN | 106083811 | A | 09 November 2016 | CN | 106083811 | B | 05 February 2019 |
| CN | 101721369 | A | 09 June 2010 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201810380857